# EUROPEAN PATENT APPLICATION

(11) **EP 2 368 967 A1**
(43) Date of publication of application: **28.09.2011**
(21) Application number: 10157179.2
(22) Date of filing: 22.03.2010
(51) Int. Cl.: C10G 3/00, C10L 1/08, C07C 9/14, C07C 9/22

(54) **Solvent composition**

(71) Applicant: Neste Oil Oyj, 02150 Espoo (FI)
(72) Inventor: Makkonen, Jaana, FI-01150, Söderkulla (FI); Kuronen, Markku, FI-02360, Espoo (FI)
(74) Representative: Kurra, Sirpa Eliina

(57) **Abstract**

The invention relates to a solvent composition containing 5 to 30 % of C₁₀-C₂₀ n-alkanes, and 70 to 95 % of C₁₀-C₂₀ iso-alkanes, by weight, said solvent composition being produced from raw materials of biological origin.

## Description

### Field of the invention

The invention relates to environmentally friendly solvent compositions of biological origin, containing paraffinic C₁₀-C₂₀ hydrocarbons, said compositions being useful for instance as technochemical and industrial solvents, drilling fluids for oil recovery, hydraulic oils, cable oils, heat transfer fluids, agricultural chemicals, fuels for heating and lightning purposes, and further, in the production and processing of polymers, and in extraction processes.

### Prior art

At present, direct or indirect reduction of detrimental impacts on nature is aimed at in nearly all technical fields. The achievement of these aims is also pushed forward by legislative actions, and thus most harmful products disappear from the market as soon as substitutes and less detrimental products are available. This is also true for solvents used in various applications in high amounts.

Solvents should not only have excellent dissolving properties, but also very low volatility, no odour and no toxic or other hazardous impacts on the well-being of mammals, fish or aquatic organisms, nor should they be accumulated in nature, or be inflammable. Accordingly, for instance the use of aromatic compounds in solvents is no longer generally desired.

US 2007260102 discloses solvents produced from starting materials of biological origin. In this publication, the production of n-paraffins by hydrogenation from vegetable oil is described, yielding C₁₀-C₁₃ n-paraffins useful as biological solvents.

WO 2007068800 discloses the oligomerization of carboxylic acids of biological origin to give compounds with longer chains, followed by hydrodeoxygenation thereof to obtain n-paraffins. These compounds may then be optionally isomerized to give corresponding branched iso-paraffins. C₁₁-C₂₆ paraffinic fraction is obtained as the product, useful as a solvent.

### Object of the invention

The object of the invention is to provide a carbon dioxide neutral solvent composition contributing to the reduction of carbon dioxide and greenhouse emissions, said composition containing paraffinic hydrocarbons.

Another object of the invention is also a solvent composition containing paraffinic hydrocarbons of biological origin wherein the carbon dioxide generated from the solvent during combustion thereof, or carbon dioxide sources released therefrom only contain carbon from organic material.

Characteristic features of the inventive solvent composition are presented in the protective claims.

### Definitions

The term "biosolvent" as used here refers to a solvent produced from starting materials of biological origin.

### General description of the invention

The present invention is directed to an aliphatic solvent composition comprising C₁₀-C₂₀ n-alkanes and isoalkanes. The solvent composition is produced from raw materials of biological origin, and thus said solvent composition is excellently useful as an environmentally friendly biosolvent. Carbon dioxide generated from the solvent during combustion, or carbon dioxide sources released therefrom only contain carbon derived from organic material.

The solvent composition has a boiling range of 180 to 340 °C, density at +15 °C of 770 to 790 kg/m³, viscosity of 2.0 to 4.0 mm²/kg, cloud point of 0 to -30 °C, flash point of at least 61 °C, said composition containing aromatic compounds no more than 1 %, by weight. The solvent composition is colourless, odourless, and chemically very stable.

The solvent composition is useful in technochemical and industrial applications and may be used as such, or as a mixture with other solvents and/or additives/aids used in the field. Moreover, drilling fluids for oil recovery, hydraulic oils, cable oils, heat transferring fluids, agricultural chemicals, fuels for heating and lightning purposes, and further, solvents for the production and processing of polymers, and in extraction processes may be mentioned as application examples.

### Detailed description of the invention

The inventors of this solvent composition found out that a solvent composition may be produced from n-alkanes and iso-alkanes of biological origin, which has several advantages over solvents of the prior art. The aliphatic solvent composition of the invention contains C₁₀-C₂₀ n-alkanes and iso-alkanes. More specifically, said solvent composition contains 5 to 30 % of C₁₀-C₂₀ n-alkanes and 70 to 95 % of C₁₀-C₂₀ iso-alkanes, preferably 10 to 20 % of C₁₀-C₂₀ n-alkanes and 80 to 90 % of C₁₀-C₂₀ iso-alkanes, all percentages being by weight. Particularly preferably, at least 70 %, by weight, of the C₁₀-C₂₀ n-alkanes are C₁₆-C₁₈ n-alkanes, and at least 70 %, by weight, of the C₁₀-C₂₀ iso-alkanes are C₁₆-C₁₈ iso-alkanes, and accordingly, most of the constituents of the solvent composition have boiling points of above 250 °C.

The solvent composition has a boiling range of 180 to 340 °C, density at +15 °C of 770 to 790 kg/m³, viscosity of 2.0 to 4.0 mm²/kg, cloud point of 0 to -30 °C, flash point of at least 61 °C, said composition containing no more than 1 %, preferably no more than 0.5 %, of aromatic compounds, no more than 0.5 % of naphthenic compounds, by weight, and no more than 5 mg/kg of sulfur. The solvent composition is colourless, odourless, and chemically very stable.

The table 1 below shows typical properties of the biosolvent composition of the invention, and the methods used for the determination thereof.

**Table 1**

| Property | Unit | Requirement | Method |
|---|---|---|---|
| Aspect, +25 °C | - | C&B (clear) | D 4176-1 |
| Sulfur | mg/kg | max 5,0 | EN ISO 20846 |
| Distillation, 95 % evaporation, by vol | °C | max 320 | EN ISO 3405 |
| 180 °C | %, by vol | max. 10 | EN ISO 3405 |
| 340 °C | %, by vol | 770...790 | EN ISO 12185 |
| Density, 15°C | kg/m³ | | EN ISO 2719 |
| Flash point | °C | min. 61 | EN ISO 3104 |
| Viscosity, 40°C | mm²/s | 2,00...4,00 | EN 23015, D 5773 |
| Cloud point | °C | 0...-30 | |
| Water | mg/kg | max 200 | EN ISO 12937 |
| Copper corrosion 3 h 50 °C | | 1 | EN ISO 2160 |
| Aromatics | | | |
| polyaromatic compounds | %, by weight | max 0,1 | NM 443, (Neste method) |
| total aromatics | %, by weight | max. 1,0 | NM 443, (Neste method) |
| Naphthenic compounds | %, by weight | max. 0,5 | NM 443, (Neste method) |
| Acid number | mg KOH/g | max. 0,01 | |

The composition of the invention may be produced from starting materials of biological origin using for instance a process first comprising a hydrodeoxygenation (HDO) step for decomposing the structure of the biological ester or triglyceride constituent, and for removing oxygen, phosphorus and sulfur compounds, concurrently hydrogenating the olefinic bonds, followed by isomerization of the product thus obtained, thus branching the hydrocarbon chain and improving the low temperature properties of the paraffin. The product may for instance fractionated to give the desired fractions.

Biological raw materials from plants, animals or fish containing fatty acids and/or fatty acid esters may be used as the starting material. The raw material may be selected from the group consisting of vegetable oils, animal fats, fish oils, and mixtures thereof. Suitable biological raw materials include rapeseed oil, canola oil, colza oil, tall oil, sunflower oil, soybeen oil, hemp oil, oilve oil, linenseed oil, mustard oil, palm oil, arachis oil, castor oil, coconut oil, animal fats such as suet, tallow, blubber, recycled alimentary fats, starting materials produced by genetic engineering, and biological starting materials produced by microbes such as algae and bacteria. Condensation products, esters, or other derivatives obtained from biological raw materials may also be used as starting materials.

In the HDO step, hydrogen gas and the biological constituent are passed to the HDO catalyst bed either in countercurrent or concurrent manner. In the HDO step, the pressure and the temperature range between 20 and 150 bar, and between 200 and 500 °C, respectively. In the HDO step, known hydrodeoxygenation catalysts may be used.

Prior to the HDO step, the biological raw material may optionally be subjected to prehydrogenation under milder conditions to avoid side reactions of the double bonds.

After the HDO step, the product is passed to the isomerization step where hydrogen gas and the biological constituent to be hydrogenated, and optionally the n-paraffin mixture are passed to the isomerization catalyst bed either concurrent or countercurrent manner.

In the isomerization step, the pressure and the temperature range between 20 and 150 bar, and between 200 and 500 °C, respectively. In the isomerization step, isomerization catalysts known as such may be typically used.

The solvent composition is produced from raw materials of biological origin, and accordingly, the solvent composition is very useful as a biosolvent. Since no fossil starting materials, but on the contrary, raw materials of biological origin from renewable resources are used in the production of the solvent composition of the invention, the total impact of the production and use on the greenhouse gas balance is lower than that of solvent compositions made of fossil raw materials. Greenhouse emissions of the solvent composition during the whole life cycle are typically lower than those of similar solvent compositions made of conventional fossil raw materials. Carbon dioxide generated from the solvent composition during combustion thereof, or carbon dioxide sources released therefrom only contain carbon from organic material.

The solvent composition has no impact on the atmosperic ozone layer, no toxic effects on fish or aquatic organisms, nor does it acccumulate in the environment.

Since the solvent composition typically contains paraffins mainly boiling at a temperature of above 250 °C, the volatility thereof is low, and accordingly, no significant amounts of detrimental emissions of volatile components are released therefrom to the environment, and moreover, the composition is classified to be a "no-VOC" product. The solvent composition has a high ignition point, and thus, the use thereof is safe. It contains minute amounts of aromatic compounds, typically clearly less than 1 %, by weight, and insignificant amounts of olefinic compounds or other reactive components. In addition, due to the fact that the solvent composition contains no more than 0.5 %, by weight, of naphthenic compounds, the composition is odourless, lacking the typical strong odour caused by naphthenes present in products based on crude oil.

The composition has a superior chemical stability, and low turbidity and freezing points, it is highly compatible with various other materials, it is non-corrosive with respect to metals, it contributes to the adjustment of viscosities of other constituents, and it has excellent wetting properties due to low surface tension. By mixing the composition to conventional solvents, harmful effects thereof may be reduced, and the proportion of constituents of biological origin may be increased.

Solvent properties of the composition is excellent, and thus it is useful in various applications as such or in combination with other solvents and/or additives/aids used in the field. Especially the paraffinic combination of C₁₀-C₂₀ n-alkanes and iso-alkanes, low volatility and high paraffin content of the composition in combination with the chemical stability, chemical inertia, low viscosity, and density make the composition particularly useful in several application. Of these, technical, techochemical and agricultural solvent applications, odourless coatings, paints, surface treatment agents, printing inks and adhesives, non-aqueous tensides, plant protecting agents, solvents for dry cleaning, wood treatment agents, cleaning and defatting agents for apparatuses of foor industry and for metals in general, aids for machining, aids for paper production, process fluids, solvents for polymer and extraction processes, and additives such as plasticizers, cleaning and polishing agents, aerosol products, finishing lubricants for textile fibers, air refresheners, animal sprays for farms, lubricating agents for pelleting of animal fodder, cosmetic products, lamp oils, and drilling fluids may be mentioned as examples. Sticker adhesives, lubricants, compositions for drilling operations at oil-fields, and compositions used in the production, application/distribution of bitumen are further examples of uses.

Majority of the constituents boiling above 250 °C, the solvent composition is particularly suitable as a component for paint industry. In paint industry, non-VOC (VOC = volatile organic compound) refers to compounds boiling above 250 °C. Solvent compositions based on crude oil typically comprises 50 % by weight of naphthenes, causing unpleasant odour. The inventive solvent composition comprises no more than 0.5 %, by weight, of naphtenes, and is thus completely odourless.

The invention is now illustrated by the embodiment described in the example, without wishing to limit the scope of the invention.

### Example

Table 2 shows the properties of a solvent composition based on crude oil, NESSOL LIAV270, produced from crude oil by first performing depletion of sulfur, followed by depletion of aromatics, and finally fractionation, as well as those of the solvent composition of the invention. The solvent composition of the invention is produced from palm oil and animal fat as raw material with a method comprising subjecting the raw material to a hydrodeoxygenation step, followed by isomerization of the product thus obtained, and finally, fractionation of the product.

**Table 2**

| The solvent composition of the invention is shown, a solvent based on crude oil serving as a control. The solvent based on crude oil is produced by first performing depletion of sulfur, followed by depletion of aromatics, and finally fractionation. | | | |
|---|---|---|---|
| PROPERTY | METHOD | NESSOL LIAV270 | INVENTION |
| n-paraffins (%, by weight) | GC-method | 22 | 13 |
| i-paraffins (%, by weight) | GC-method | 31 | 87 |
| Naphthenes (%, by weight) | GC-method | 47 | <0,2 |
| C/H ratio | GC-method | 5.79 | 5.61 |
| Density + 15 °C (kg/m³) | ENISO 12185 | 814 | 780 |
| Kinematic viscosity +20°C (mm²/s) | ENISO 3104 | 3.5 | 4.7 |
| Flash point °C | ENISO 2719 | 102 | 99 |

## Claims

1. Solvent composition, **characterized in that** the solvent composition contains 5 to 30 % of C₁₀-C₂₀ n-alkanes, and 70 to 95 % of C₁₀-C₂₀ iso-alkanes, by weight, said solvent composition being produced from raw materials of biological origin.

2. Solvent composition according to claim 1, **characterized in that** the solvent composition contains 10 to 20 % of C₁₀-C₂₀ n-alkanes, and 80 to 90 % of C₁₀-C₂₀ iso-alkanes, by weight.

3. Solvent composition according to claim 1 or 2, **characterized in that** at least 70 %, by weight, of the C₁₀-C₂₀ n-alkanes are C₁₆-C₁₈ n-alkanes, and at least 70 %, by weight, of the C₁₀-C₂₀ iso-alkanes are C₁₆-C₁₈ iso-alkanes.

4. Solvent composition according to any of the claims 1 to 3, **characterized in that** the solvent composition has a boiling range of 180 to 340 °C, density at + 15 °C of 770 to 790 kg/m³, viscosity of 2.0 to 4.0 mm²/kg, cloud point of 0 to -30 °C, flash point of at least 61 °C, and said composition contains no more than 1 % of aromatic compounds, and less than 0.5 % of naphthenic compounds, by weight.

5. Solvent composition according to any of the claims 1 to 4, **characterized in that** the solvent composition is a technochemical solvent, an industrial solvents, an agricultural solvent, a fuel or a constituent thereof or in drilling fluids for oil recovery, hydraulic oils, cable oils, or heat transfer fluids.

6. Solvent composition according to any of the claims 1 to 5, **characterized in that** the solvent composition is a constituent in a coating, paint, surface treatment agent, printing ink, or adhesive.
